(19) Europäisches Patentamt
European Patent Office
Office européen des brevets

(11) Numéro de publication : **0 151 140**
**B1**

(12) # FASCICULE DE BREVET EUROPÉEN

(45) Date de publication du fascicule du brevet : **03.12.86**

(51) Int. Cl.⁴ : **C 07 D239/42**

(21) Numéro de dépôt : **84902455.9**

(22) Date de dépôt : **21.06.84**

(86) Numéro de dépôt international : **PCT/FR 84/00156**

(87) Numéro de publication internationale : **WO/8500168 (17.01.85 Gazette 85/02)**

(54) **SEL DE 2-PIPERAZINOPYRIMIDINE, PROCEDE POUR SA PREPARATION ET COMPOSITIONS PHARMACEUTIQUES EN CONTENANT.**

(30) Priorité : **24.06.83 FR 8310525**

(43) Date de publication de la demande : **14.08.85 Bulletin 85/33**

(45) Mention de la délivrance du brevet : **03.12.86 Bulletin 86/49**

(84) Etats contractants désignés : **AT BE CH DE FR GB LI LU NL SE**

(56) Documents cités : **Journal of Chromatography, vol. 252, décembre 1982, Elsevier (Amsterdam, NL) S. Caccia et al.: "Identification and quantitation of 1-(2-pyrimidinyl) piperazine, an active metabolite of the anxiolytic agent buspirone, in rat plasma and brain", voir page 310 (II), page 312, tableau I; page 314**

(73) Titulaire : **SANOFI**
**40, Avenue George V**
**F-75008 Paris (FR)**

(72) Inventeur : **CRISAFULLI, Emilio**
**Viale San Gimignano 12**
**I-20146 Milano (IT)**
Inventeur : **FRIGERIO, Marco**
**Via Carlo Poma 28**
**Mantova (IT)**
Inventeur : **KAN, Jean Paul**
**7 rue du Vallon Claplers**
**F-34170 Castelneau-le-Lez (FR)**

(74) Mandataire : **Gillard, Marie-Louise et al**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe

**EP 0 151 140 B1**

**Description**

La présente invention concerne un nouveau sel de 2-pipérazinopyrimidine ayant une action psychotrope dopaminergique.

Plus particulièrement, l'invention se réfère au sel de 2-(1-pipérazinyl)pyrimidine avec l'acide 2-naphtalène-sulfonique, à un procédé pour sa préparation et à des compositions pharmaceutiques le renfermant en tant que principe actif.

La 2-(1-pipérazinyl)pyrimidine est, en tant que base libre, un composé bien connu en littérature ; il est utilisé en tant qu'intermédiaire de synthèse.

Il a été testé parmi toute une série de composés dont aucun n'a montré d'activité analgésique ou antifilariale (H. W. Stewart et al., J. Org. Chem. 1953, 18, 1478-1483).

Ce composé a été isolé comme métabolite actif de l'anxiolytique buspirone (J. Chromatography 1982, *252*, 310-314).

On a maintenant trouvé que le sel de la 2-(1-pipérazinyl)pyrimidine avec l'acide 2-naphtalènesulfonique possède une très bonne activité psychotrope avec un mécanisme d'action dopaminergique, notamment une activité antipsychotique, antidépressive et tranquillisante-sédative.

Il a été également trouvé d'une façon surprenante que le sel de la 2-(1-pipérazinyl)pyrimidine avec l'acide 2-naphtalènesulfonique est beaucoup plus actif en sens absolu que la base libre correspondante et son chlorhydrate et que cette activité très élevée est de longue durée et augmente avec le temps.

Ainsi la présente invention a pour objet, selon un de ses aspects, le sel de la 2-(1-pipérazinyl)pyrimidine avec l'acide 2-naphtalènesulfonique de formule

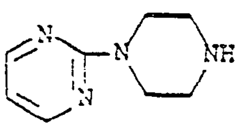 , 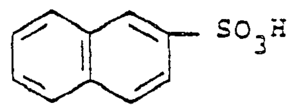

ci-après indiqué « 2-(1-pipérazinyl)pyrimidine napsilate ».

Le composé de la présente invention est préparé, selon un autre aspect de la présente invention, par un procédé de salification, caractérisé en ce qu'on traite la 2-(1-pipérazinyl)pyrimidine avec une quantité équimoléculaire d'acide 2-naphtalènesulfonique, dans un solvant organique ou organique aqueux.

Comme solvant organique on utilise de préférence un alcool, tel que l'isopropanol, ou l'acétone.

Le sel ainsi obtenu est isolé selon les techniques classiques par simple filtration et cristallisation éventuelle.

La salification peut être conduite sur une 2-(1-pipérazinyl)pyrimidine à l'état brut telle qu'obtenue de la réaction entre la 2-chloropyrimidine et la pipérazine selon des modes opératoires connus.

Selon un mode opératoire préféré, la 2-(1-pipérazinyl)pyrimidine de départ est utilisée sous forme de chlorhydrate et l'acide 2-naphtalènesulfonique est utilisé sous forme d'un sel alcalin, de sodium de préférence. La salification se produit à une température de 50-70 °C dans un solvant organique aqueux, tel que l'acétone contenant 5 à 20 % d'eau.

L'activité antidépressive du composé de l'invention a été évaluée dans le test de l'antagonisme vis-à-vis de l'installation de la catalepsie provoquée par la prochlorpérazine (K. Bizière et al., Arzneimittel Forschung, 1982, *32* (II), 824-831).

Le 2-(1-pipérazinyl)pyrimidine napsilate et la base libre correspondante, comme produit de référence, ont été administrés par voie orale à des groupes de 10 rats mâles Wistar pesant 220-240 g ; des animaux de contrôle ont été en même temps traités par du sérum physiologique. Une heure après, les animaux ont reçu, par voie sous-cutanée, de la prochlorpérazine à une dose de 10 mg/kg. Cinq heures après cette administration, le nombre d'animaux cataleptiques a été estimé par le test du bouchon. Ce test consiste à positionner les animaux de telle sorte qu'ils puissent maintenir pendant au moins 20 secondes leurs pattes en appui sur une colonne formée de trois bouchons de liège superposés (hauteur totale 11 cm). Les performances réalisées pour chaque groupe d'animaux ont été comparées à celles du groupe d'animaux témoins n'ayant reçu que le véhicule et la prochlorpérazine.

Le tableau I ci-après montre la DE50 des composés testés.

(Voir Tableau I page 3)

De ce tableau il ressort que le 2-(1-pipérazinyl)-pyrimidine napsilate de la présente invention est, par voie orale, environ 4 fois plus actif que la base libre correspondante dans le test de la catalepsie provoquée par la prochlorpérazine, révélateur d'une activité antidépressive.

Le mécanisme dopaminergique du composé de l'invention a été étudié en comparaison avec la base libre correspondante et son chlorhydrate par l'analyse du comportement de rotation chez la souris après lésion unilatérale du striatum (P. Protais et al. J. Pharmacol. 1976, 7, 251-255).

Des souris femelles Charles River CD1 pesant de 20 à 24 g ont préalablement fait l'objet d'une lésion

Tableau I

| Composé | Antagonisme vis-à-vis de l'installation de la catalepsie provoquée par la Prochlorpérazine DE50 µmol/kg |
|---|---|
| 2-(1-pipérazinyl)-pyrimidine napsilate | 5,7 (5,0 - 6,4) |
| 2-(1-pipérazinyl)-pyrimidine base | 24 (12 - 45) |

unilatérale du striatum par injection stéréotaxique de 6-hydroxydopamine à raison de 8 µg par animal. Une semaine après cette opération, le 2-(1-pipérazinyl)pyrimidine napsilate, le 2-(1-pipérazinyl)pyrimidine chlorhydrate et la base libre ont été administrés par voie orale à des groupes de 6 souris à une dose correspondant à 0,15 mg/kg de base libre. Le nombre de rotations a été évalué, pendant 2 minutes, 1 heure après l'administration des trois produits. Les rotations ipsilatérales à la lésion ont été comptées positivement, celles contralatérales ont été comptées négativement. La somme algébrique des rotations pour chaque groupe d'animaux traités a été comparée à celle du groupe d'animaux témoins n'ayant reçu que le véhicule (sérum physiologique).

Le Tableau II ci-dessous montre la variation pour cent, par rapport aux animaux témoins, des rotations.

Tableau II

| Composé | Dose (mg/kg) | Variation % |
|---|---|---|
| 2-(1-pipérazinyl)pyrimidine base | 0,15 | - 94 ** |
| 2-(1-pipérazinyl)pyrimidine chlorhydrate | 0,18 | - 29 ns |
| 2-(1-pipérazinyl)pyrimidine napsilate | 0,34 | -117 ** |

** significatif, $p < 0,01$ — Test "t" de Student
ns: non significatif

De ce tableau il ressort que la base réduit d'une façon significative la somme algébrique des rotations ipsilatérales et contralatérales à la lésion, alors que le chlorhydrate n'a pas d'effet significatif. En plus, le napsilate non seulement réduit la somme algébrique ci-dessus, mais il montre une variation négative supérieure à 100 %, ce qui démontre que le nombre des rotations contralatérales à la lésion est plus grand que le nombre des rotations ipsilatérales à la lésion. Le napsilate possède donc une activité dopaminergique plus élevée que celle de la base libre tandis que le chlorhydrate dans ces conditions est presqu'inactif.

L'action dopaminergique du composé de la présente invention a été également évaluée dans le temps en comparaison avec la base libre correspondante et son chlorhydrate.

Le Tableau III ci-dessous montre la variation pour cent des rotations ipsilatérales et contralatérales à

la lésion 60, 180 et 360 minutes après l'administration des trois produits par voie orale, à une dose correspondant à 0,15 mg/kg de base libre, par rapport aux témoins n'ayant reçu que le véhicule.

Tableau III

| Composé | Variation % après minutes: | | |
|---|---|---|---|
| | 60 | 180 | 360 |
| 2-(1-pipérazinyl)-pyrimidine base | - 94 ** | - 62 ** | -60 ** |
| 2-(1-pipérazinyl)pyrimidine chlorhydrate | - 29 ns | - 28 ns | -22 ns |
| 2-(1-pipérazinyl)-pyrimidine napsilate | -117 ** | -126 ** | -147 ** |

** significatif $p < 0{,}01$ Test "t" de Student
ns: non significatif

De ce tableau il ressort que le chlorhydrate de 2-(1-pipérazinyl)pyrimidine est inactif aussi dans le temps et que la base libre montre une tendance à la diminution de l'activité dans le temps. Le 2-(1-pipérazinyl)pyrimidine napsilate de la présente invention non seulement est beaucoup plus puissant que les produits de référence, mais, en plus, son activité augmente significativement avec le temps.

L'activité psychotrope dopaminergique du composé de la présente invention et sa faible toxicité le rendent utile comme médicament.

Ainsi, selon un autre de ses aspects, la présente invention concerne des compositions pharmaceutiques renfermant le 2-(1-pipérazinyl)pyrimidine napsilate en tant qu'ingrédient actif.

Dans les compositions pharmaceutiques de la présente invention pour l'administration orale, parentérale, sublinguale, transdermique ou rectale, le 2-(1-pipérazinyl)-pyrimidine napsilate, utilisé comme ingrédient actif, peut être administré sous formes unitaires d'administration, en mélange avec des supports pharmaceutiques classiques, aux animaux et aux êtres humains pour le traitement des troubles de l'humeur ou du comportement, notamment dans le cas de psychose, dépressions, ainsi que des états anxieux et des insomnies. Parmi les formes unitaires d'administration appropriées, il y a les formes par voie orale telles que les comprimés, les gélules, les poudres, les granules et les solutions ou suspensions orales et les suppositoires pour une administration rectale.

Afin d'obtenir l'effet psychotrope désiré, la dose de principe actif peut varier entre 0,1 et 100 mg par kg de poids du corps et par jour.

Chaque dose unitaire peut contenir de 1 à 300 mg d'ingrédient actif en combinaison avec un support pharmaceutique. Cette dose unitaire peut être administrée 1 à 4 fois par jour pour le traitement des troubles de l'humeur ou du comportement.

Les exemples suivants illustrent l'invention sans toutefois la limiter.

Exemple 1

A une solution de 3 g (0,015 mol) de chlorhydrate de 2-(1-pipérazinyl)pyrimidine dans 12 ml d'eau chauffée à 50-60 °C on ajoute une solution de 3,44 g (0,015 mol) de sel de sodium de l'acide 2-naphtalènesulfonique dans 120 ml d'acétone et 12 ml d'eau chauffée au préalable à la température ci-dessus. Après refroidissement et filtration, on obtient 4,6 g (82 %) de 2-(1-pipérazinyl)-pyrimidine napsilate pur ; p. f. 218-221 °C. Analyse : S total : Calc. 8,61 % ; Trouv. 8,51 %.

La structure a été confirmée par les spectres infrarouge (IR) ultraviolet (UV) et de résonance magnétique nucléaire (RMN).

Exemple 2

On chauffe au reflux pendant 18 heures un mélange de 3,2 g de 2-chloropyrimidine et de 12,1 g de

pipérazine anhydre dans 100 ml d'éthanol absolu, puis on évapore à fond sous pression réduite et à chaud. On reprend le résidu avec 400 ml d'éther diéthylique, on lave la solution ainsi obtenue d'abord avec 10 ml d'une solution aqueuse d'hydroxyde de sodium 1 : 1, puis avec 20 ml d'eau. On sèche la solution organique sur du sulfate de sodium anhydre, on la filtre et on l'évapore à sec sous pression réduite. On dissout l'huile ainsi obtenue dans 30 ml d'isopropanol et on ajoute à la solution 4,38 g d'acide 2-naphtalènesulfonique dissous dans 30 ml d'isopropanol à 60 °C environ. De la solution ainsi obtenue cristallise le produit qui est filtré et séché. On obtient le 2-(1-pipérazinyl)pyrimidine napsilate identique au produit de l'Exemple 1.

Exemple 3

On prépare des gélules à base de 2-(1-pipérazinyl)-pyrimidine napsilate ayant la composition suivante :

| | |
|---|---|
| principe actif | 15 mg |
| lactose | 120 mg |
| stéarate de magnésium | 5 mg |

en mélangeant intimement des charges des ingrédients ci-dessus et en versant le mélange dans des gélules de gélatine dure.

Exemple 4

On prépare des comprimés à base de 2-(1-pipérazinyl)-pyrimidine napsilate ayant la composition suivante :

| | |
|---|---|
| principe actif | 20 mg |
| lactose | 100 mg |
| cellulose microcristalline | 30 mg |
| amidon de maïs séché | 40 mg |
| stéarate de magnésium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

Exemple 5

En opérant comme décrit dans l'Exemple 4 ci-dessus, on prépare des comprimés ayant la composition suivante :

| | |
|---|---|
| principe actif | 50 mg |
| lactose | 95 mg |
| amidon de maïs | 100 mg |
| talc | 4,5 mg |
| stéarate de magnésium | 0,5 mg |

Exemple 6

On prépare des suppositoires à base de 2-(1-pipérazinyl)pyrimidine napsilate ayant la composition suivante :

| | |
|---|---|
| principe actif | 50 mg |
| lactose | 250 mg |
| masse pour suppositoires | q.s.p. 1,7 g |

On mélange la substance active avec le lactose et on met le mélange uniformément en suspension dans la masse pour suppositoires fondue. On verse la suspension dans des moules refroidis pour former des suppositoires d'un poids de 1,7 g.

**Revendications** (pour les Etats contractants : BE, CH, DE, FR, GB, LI, LU, NL, SE)

1. Sel de la 2-(1-pipérazinyl)pyrimidine avec l'acide 2-naphtalènesulfonique de formule

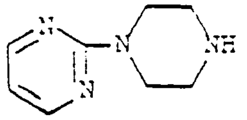 , 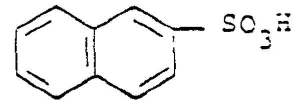

2. Procédé pour la préparation de la 2-(1-pipérazinyl)pyrimidine napsilate selon la revendication 1, caractérisé en ce que l'on traite la 2-(1-pipérazinyl)-pyrimidine avec une quantité équimoléculaire de l'acide 2-naphtalènesulfonique dans un solvant organique ou organique aqueux.

3. Procédé selon la revendication 2, caractérisé en ce que la salification est conduite sur une 2-(1-pipérazinyl)pyrimidine brute, telle qu'obtenue de la réaction entre la 2-chloropyrimidine et la pipérazine.

4. Procédé selon la revendication 2, caractérisé en ce que la 2-(1-pipérazinyl)pyrimidine de départ est utilisée sous forme de chlorhydrate et l'acide 2-naphtalènesulfonique est utilisé sous forme d'un seul alcalin.

5. Procédé selon la revendication 4, caractérisé en ce que la salification est conduite à une température de 50-70 °C dans de l'acétone contenant 5 à 20 % d'eau.

6. Composition pharmaceutique à action psychotrope dopaminergique renfermant, en tant qu'ingrédient actif, le composé selon la revendication 1.

7. Composition pharmaceutique selon la revendication 6, sous forme de dosage unitaire.

8. Composition pharmaceutique selon la revendication 7, caractérisée en ce qu'elle contient, pour chaque unité de dosage, de 1 à 300 mg d'ingrédient actif en mélange avec un excipient pharmaceutique.

**Revendications** (pour l'Etat contractant AT)

1. Procédé de préparation du sel de la 2-(1-pipérazinyl) pyrimidine avec l'acide 2-naphtalènesulfonique, de formule

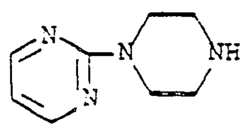 , 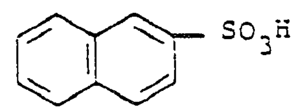

caractérisé en ce que l'on traite la 2-(1-pipérazinyl)pyrimidine avec une quantité équimoléculaire de l'acide 2-naphtalènesulfonique dans un solvant organique ou organique aqueux.

2. Procédé selon la revendication 1, caractérisé en ce que la salification est conduite sur une 2-(1-pipérazinyl)pyrimidine brute, telle qu'obtenue de la réaction entre la 2-chloropyrimidine et la pipérazine.

3. Procédé selon la revendication 1, caractérisé en ce que la 2-(1-pipérazinyl)pyrimidine de départ est utilisée sous forme de chlorhydrate et l'acide 2-naphtalènesulfonique est utilisé sous forme d'un sel alcalin.

4. Procédé selon la revendication 3, caractérisé en ce que la salification est conduite à une température de 50-70 °C dans de l'acétone contenant 5 à 20 % d'eau.

5. Utilisation du sel obtenu selon l'une quelconque des revendications 1 à 4, pour la préparation de compositions pharmaceutiques à action psychotrope.

6. Utilisation du sel obtenu selon l'une quelconque des revendications 1 à 4, pour la préparation de compositions pharmaceutiques à action psychotrope, sous forme de dosage unitaire.

7. Utilisation selon la revendication 6 du sel obtenu selon l'une quelconque des revendications 1 à 4, lesdites compositions pharmaceutiques contenant, pour chaque unité de dosage de 1 à 300 mg dudit sel en mélange avec un excipient pharmaceutique.

**Claims** (for the Contracting States : BE, CH, DE, FR, GB, LI, LU, NL, SE)

1. The 2-(1-piperazinyl)pyrimidine salt with the 2-naphtalenesulfonic acid of formula

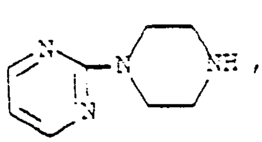 , 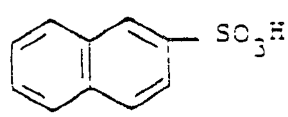

2. A process for the preparation of 2-(1-piperazinyl)pyrimidine napsilate according to claim 1, characterized in that it comprises treating 2-(1-piperazinyl)pyrimidine with an equimolecular amount of 2-naphtalenesulfonic acid in an organic or aqueous organic solvent.

3. A process according to claim 2, characterized in that the salification is carried out on a crude 2-(1-piperazinyl)pyrimidine, as obtained from the reaction of 2-chloropyrimidine with piperazine.

4. A process according to claim 2, characterized in that the starting 2-(1-piperazinyl)pyrimidine is used as hydrochloride and the 2-naphtalenesulfonic acid is used as an alkaline salt.

5. A process according to claim 4, characterized in that the salification is carried out at a temperature of 50-70 °C in acetone containing 5 to 20 % of water.

6. A pharmaceutical composition having dopaminergic psychotropic action containing, as active ingredient, the compound according to claim 1.

7. A pharmaceutical composition according to claim 6, which is in dosage unit form.

8. A pharmaceutical composition according to claim 7, characterized in that it contains from 1 to 300 mg of active ingredient per dosage unit in admixture with a pharmaceutical carrier.

**Claims** (for the Contracting State AT)

1. Process for preparing 2-(1-piperazinyl)pyrimidine salt with the 2-naphtalenesulfonic acid of formula

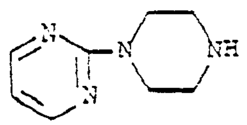 , 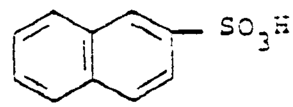

characterized in that it comprises treating 2-(1-piperazinyl)pyrimidine with an equimolecular amount of 2-naphtalenesulfonic acid in an organic or aqueous organic solvent.

2. A process according to claim 1, characterized in that the salification is carried out on a crude 2-(1-piperazinyl)pyrimidine, as obtained from the reaction of 2-chloropyrimidine with piperazine.

3. A process according to claim 1, characterized in that the starting 2-(1-piperazinyl)pyrimidine is used as hydrochloride and the 2-naphtalenesulfonic acid is used as an alkaline salt.

4. A process according to claim 3, characterized in that the salification is carried out at a temperature of 50-70 °C in acetone containing 5 to 20 % of water.

5. Use of the salt obtained according to any one of claims 1 to 4, for the preparation of pharmaceutical compositions having psychotropic action.

6. Use of the salt obtained according to any one of claims 1 to 4 for the preparation of pharmaceutical compositions having psychotropic action, in dosage unit form.

7. Use according to claim 6 of the salt obtained according to any one of claims 1 to 4, said pharmaceutical compositions containing per dosage unit, from 1 to 300 mg of said salt in admixture with a pharmaceutical carrier.

**Patentansprüche** (für die Vertragsstaaten : BE, CH, DE, FR, GB, LI, LU, NL, SE)

1. Salz von 2-(Piperazin-1-yl)-pyrimidin mit 2-Naphthalinsulfonsäure der Formel

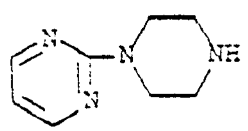 , 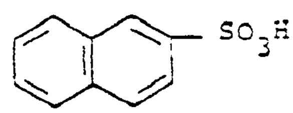

2. Verfahren zur Herstellung von 2-(Piperazin-1-yl)-pyrimidin-napsilat nach Anspruch 1, dadurch gekennzeichnet, daß man 2-(Piperazin-1-yl)-pyrimidin mit einer äquimolaren Menge von 2-Naphthalinsulfonsäure in einem organischen oder organisch-wässerigen Lösungsmittel behandelt.

3. Verfahren nach Anspruch 2, dadurch gekennzeichnet, daß die Salzbildung an rohem 2-(Piperazin-1-yl)-pyrimidin, wie es aus der Reaktion zwischen 2-Chlorpyrimidin und Piperazin erhalten wird, durchgeführt wird.

4. Verfahren nach Anspruch 2, dadurch gekennzeichnet, daß das Ausgangs-2-(Piperazin-1-yl)-pyrimidin in Form des Hydrochlorids und die 2-Naphthalinsulfonsäure in Form eines Alkalisalzes verwendet werden.

5. Verfahren nach Anspruch 4, dadurch gekennzeichnet, daß die Salzbildung bei einer Temperatur von 50 bis 70 °C in 5 bis 20 % Wasser enthaltendem Aceton durchgeführt wird.

6. Pharmazeutische Zusammensetzung mit psychotroper dopaminergischer Wirkung, die als aktives Ingrediens die Verbindung nach Anspruch 1 enthält.

7. Pharmazeutische Zusammensetzung nach Anspruch 6 in Einheitsdosisform.

8. Pharmazeutische Zusammensetzung nach Anspruch 7, dadurch gekennzeichnet, daß sie pro Dosiseinheit 1 bis 300 mg aktives Ingrediens in Mischung mit einem pharmazeutischen Exzipienten enthält.

**Patentansprüche** (für den Vertragsstaat AT)

1. Verfahren zur Herstellung des Salzes von 2-(Piperazin-1-yl)-pyrimidin mit 2-Naphthalin-sulfonsäure der Formel

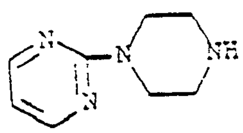 , 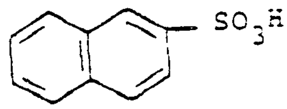

dadurch gekennzeichnet, daß man 2-(Piperazin-1-yl)-pyrimidin mit einer äquimolaren Menge von 2-Naphthalinsulfonsäure in einem organischen oder organisch-wässerigen Lösungsmittel behandelt.

2. Verfahren nach Anspruch 1, dadurch gekennzeichnet, daß die Salzbildung an röhem 2-(Piperazin-1-yl)-pyrimidin, wie es aus der Reaktion zwischen 2-Chlorpyrimidin und Piperazin erhalten wird, durchgeführt wird.

3. Verfahren nach Anspruch 1, dadurch gekennzeichnet, daß das Ausgangs-2-(Piperazin-1-yl)-pyrimidin in Form des Hydrochlorids und die 2-Naphthalinsulfonsäure in Form eines Alkalisalzes verwendet werden.

4. Verfahren nach Anspruch 3, dadurch gekennzeichnet, daß die Salzbildung bei einer Temperatur von 50 bis 70 °C in 5 bis 20 % Wasser enthaltendem Aceton durchgeführt wird.

5. Verwendung des nach einem der Ansprüche 1 bis 4 erhaltenen Salzes zur Herstellung von pharmazeutischen Zusammensetzungen mit psychotroper Wirkung.

6. Verwendung des nach einem der Ansprüche 1 bis 4 erhaltenen Salzes zur Herstellung von pharmazeutischen Zusammensetzungen mit psychotroper Wirkung in Einheitsdosisform.

7. Verwendung des nach einem der Ansprüche 1 bis 4 erhaltenen Salzes nach Anspruch 6, welche pharmazeutische Zusammensetzungen pro Dosiseinheit 1 bis 300 mg des Salzes in Mischung mit einem pharmazeutischen Exzipienten enthalten.